Europäisches Patentamt

European Patent Office

Office européen des brevets

(19)

(11) Numéro de publication: **0 127 551**
**B1**

## FASCICULE DE BREVET EUROPÉEN

(12)

(45) Date de publication du fascicule du brevet:
08.04.87

(51) Int. Cl.⁴: **C 07 C 46/00,** C 07 C 50/18

(21) Numéro de dépôt: **84420075.8**

(22) Date de dépôt: **20.04.84**

(54) **Procédé de cyclisation de l'acide orthobenzoylbenzoique.**

(30) Priorité: **03.05.83 FR 8307319**
**05.07.83 FR 8311150**

(43) Date de publication de la demande:
**05.12.84 Bulletin 84/49**

(45) Mention de la délivrance du brevet:
**08.04.87 Bulletin 87/15**

(84) Etats contractants désignés:
**BE CH DE FR GB IT LI NL SE**

(56) Documents cité:
**DE-A-533 465**
**DE-A-2 633 458**
**FR-A-2 314 913**
**US-A-2 401 225**

**Le dossier contient des informations techniques présentées postérieurement au dépôt de la demande et ne figurant pas dans le présent fascicule.**

(73) Titulaire: **ATOCHEM, 4 & 8, Cours Michelet La Défense 10, F-92800 Puteaux (FR)**

(72) Inventeur: **Devic, Michel, 27 Chemin des Fonds, F-69110 Ste- Foy- lès- Lyon (FR)**

EP 0 127 551 B1

## Description

La présente invention concerne un procédé de cyclisation de l'acide orthobenzoylbenzoïque (acide OBB) en présence de terre décolorante super-active, pour obtenir de l'anthraquinone.

Il est connu de cycliser l'acide OBB en anthraquinone par chauffage de ce composé en présence d'acide sulfurique concentré ou d'un oléum. Une telle méthode est par exemple rapportée par Arthur I.VOGEL dans "Practical Organic Chemistry", 3ème édition, page 740. L'acide sulfurique joue le rôle de catalyseur et de solvant de la réaction.

L'acide sulfurique concentré peut être remplacé par de l'acide phosphorique concentré ou du pentoxyde de phosphore.

Dans chacune des méthodes citées ci-dessus, la grande quantité de réactif mise en jeu grève l'économie du procédé correspondant et crée d'importants problèmes de pollution dûs aux rejets acides.

La demande de brevet japonais publiée n° 49.7260/74 propose de travailler avec une quantité moindre d'acide sulfurique. Mais il faut alors opérer sous vide et à une température élevée, supérieure à 300°C. Ceci implique des risques opératoires et un coût élevé de l'appareillage dû à la corrosion inhérente aux conditions de réalisation d'un tel procédé. De plus, la récupération par sublimation de l'anthraquinone formée ne peut être effectuée qu'après neutralisation de l'acide sulfurique par le carbonate de sodium.

Le brevet des Etats-Unis d'Amérique n° 2 842 562 décrit un procédé permettant encore de n'employer qu'une quantité réduite d'acide sulfurique. La cyclisation de l'acide OBB a lieu alors à 260°C en présence d'un tiers-solvant comme le trichlorobenzène, l'eau formée au cours de la réaction étant éliminée par distillation sous forme d'azéotrope avec le tiers-solvant. L'emploi de ce dernier complique l'appareillage et rend peu économique la récupération de l'anthraquinone qui doit être séparée du solvant par entraînement à la vapeur de celui-ci.

Le brevet des Etats-Unis d'Amérique n° 2 174 118 propose l'acide fluorhydrique comme catalyseur de cyclisation de l'acide OBB. L'emploi d'un tel réactif entraîne des inconvénients majeurs dûs à sa nature même: corrosion par la combinaison eau-acide fluorhydrique formée au cours de l'opération, récupération difficile de l'acide fluorhydrique par deshydratation de cette même combinaison, enfin nécessité d'opérer la cyclisation de l'acide OBB sous pression.

Le brevet des Etats Unis d'Amérique n° 4 304 727 décrit un procédé dans lequel est mise en oeuvre, comme catalyseur de cyclisation, une résine perfluorée en suspension dans un solvant organique inerte. Si la récupération du catalyseur est ainsi facilitée, la conversion de l'acide OBB et le rendement en anthraquinone sont faibles, par exemple égaux respectivement à 60 % et 78 %. Il faut de plus procéder à la séparation du solvant organique de l'anthraquinone.

La demande de brevet japonais publiée n° 49.6240/74 préconise de cycliser l'acide OBB par chauffage à 360°C en présence de terre activée. Une telle façon d'opérer présente des inconvénients rendant coûteux son exploitation industrielle: nécessité d'opérer sous vide, vitesse lente de cyclisation, nécessité d'un mélange intime de l'acide OBB et de la terre activée, faible rendement pratique par suite de l'absorption de près de un quart de l'anthraquinone formée par la terre activée. La récupération de l'anthraquinone ainsi absorbée nécessiterait des opérations coûteuses d'extraction par un solvant organique et d'évaporation de ce dernier.

Le certificat d'utilité français n° 76.18056, publié sous le numéro 2 314 913, décrit la cyclisation de l'acide OBB en présence de composés oxygénés de l'aluminium et du silicium, de préférence par chauffage de l'acide OBB et de silicoaluminate pulvérulent. Lorsque le protédé est conduit en suspension dans un solvant organique, l'élimination du solvant puis du catalyseur est compliquée et coûteuse. Lorsqu'il est conduit dans un lit fixe ou dans un lit tourbillonnaire on risque, dans le premier cas, un encrassement rapide du catalyseur, dans le deuxième cas, de se heurter à une séparation délicate des fines particules de catalyseur de l'anthraquinone. Les rendements de cyclisation les meilleurs sont de plus atteints lorsque celle-ci est effectuée sous vide.

Un premier objet de l'invention est un procédé qui permet de pallier les inconvénients signalés des procédés connus.

Il consiste à effectuer la cyclisation de l'acide OBB par chauffage de celui-ci à une température comprise entre 300°C et 400°C à pression atmosphérique, en présence d'une argile de type bentonite ayant été soumise d'une façon connue à l'action d'un acide minéral puis lavée à l'eau de manière à ce que la teneur en acide, exprimée en acide chlorhydrique, n'excède pas 0,1 %, et enfin séchée à une température au plus égale à 120°C.

De telles argiles traitées sont couramment dénommées terres décolorantes super-actives et sont ordinairement utilisées dans l'industrie pour le traitement décolorant et la purification d'huiles et de graisses.

Le procédé selon l'invention permet d'opérer la cyclisation de l'acide OBB sans qu'il soit nécessaire de procéder à un mélange intime de ce composé avec la terre décolorante super-active.

Une durée de l'opération généralement comprise entre 5 et 120 minutes, le plus souvent entre 5 et 60 minutes, suffit pour que le rendement de cyclisation soit égal ou supérieur à 90%.

Une température supérieure à 400°C accélère la vitesse de cyclisation de l'acide OBB mais provoque la formation d'une quantité de benzophénone rapidement excessive.

Une température inférieure à 300°C exige une durée de réaction très vite trop importante pour rester compatible avec une réalisation économique du procédé.

La quantité de terre décolorante super-active est le plus souvent comprise entre 0,5 et 5 parties en poids, de préférence entre 1 et 3 parties en poids par partie d'acide OBB.

Parmi les terres décolorantes super-actives convenant à la réalisation de l'invention, celles qui sont préférées ont une composition pondérale comprise dans les limites suivantes: $SiO_2$ 70 % à 75 %, $Al_2O_3$ 10 % à 20 %, $Fe_2O_3$ 3 % à 5 MgO 1 % à 3 %, CaO 0,5 % à 2 %, matières éliminables par calcination 5 % à 10 %. Le pH d'une suspension aqueuse à 10 % de terre décolorante superactive de cette nature est de 4 environ. La surface spécifique de tels composés qui se présentent sous forme de grains de dimension le plus souvent inférieure à 150µm, est comprise entre 150 m²/g et 200 m²/g environ.

Il est par exemple possible d'utiliser une terre décolorante super-active, commercialisée, sous la marque "TONSIL", par la Société SÜD-CHEMIE A.G.

Il est possible de réaliser le procédé de l'invention en disposant simplement une couche de terre décolorante super-active sur une couche d'acide OBB. L'épaisseur de la couche de terre décolorante super-active doit être telle qu'une diffusion rapide de l'anthraquinone formée lors du chauffage soit permise. L'anthraquinone qui se sépare du milieu réactionnel par sublimation peut être recueillie par condensation selon une technique connue. On peut par exemple recueillir l'anthraquinone sur une surface dont la température est comprise entre 100° C et 150° C de manière à éviter la condensation de l'eau et des impuretés organiques volatiles. L'anthraquinone recueillie a ainsi un haut degré de pureté.

Le procédé selon l'invention peut être conduit en discontinu ou en continu. Un exemple de réalisation continu consiste à déposer, en une extrémité d'une bande transporteuse en acier inoxydable se déplaçant horizontalement à vitesse constante, un film d'acide OBB à partir d'acide OBB fondu, puis de déposer sur ce film une couche de la terre décolorante super-active, la bande transporteuse effectuant ensuite un parcours durant lequel les deux couches superposées sont portées à une température comprise entre 300° C et 400° C et l'anthraquinone formée se sublime et se condense par exemple sur la surface d'une bande transporteuse secondaire maintenue à une température comprise entre 100° C et 150° C et se déplaçant au-dessus de la bande transporteuse principale et parallèlement à elle. La terre décolorante super-active usagée est évacuée à l'extrémité de la bande transporteuse principale opposée à celle à laquelle est déposée l'acide OBB et peut subir un traitement de régénération en vue de son recyclage dans le procédé.

L'acide OBB contient généralement, comme impuretés, de l'acide phtalique et de l'anhydride phtalique lorsqu'il est préparé, comme c'est le cas le plus fréquent dans l'industrie, par condensation de l'anhydride phtalique avec le benzène selon par exemple le procédé décrit dans le brevet français n° 2 496 097.

La concentration d'acide phtalique dans l'acide OBB, comme d'ailleurs celle d'anhydride phtalique, peut atteindre 10 % en poids.

Un second objet de l'invertion est un procédé pour séparer l'acide phtalique et l'anhydride phtalique de l'acide OBB.

Il consiste à cycliser l'acide phtalique contenu dans l'acide OBB en anhydride phtalique qui se sépare alors du milieu réactionnel par sublimation conjointement à celui initialement présent, le cas échéant, dans l'acide OBB, en présence d'une terre décolorante superactive telle que définie pour la cyclisation de l'acide OBB, au cours d'une étape de chauffage à une température comprise préférentiellement entre 200° C et 250° C avant lieu au cours de la montée en température en vue de la cyclisation ultérieure de l'acide OBB en présence de la même terre décolorante super-active.

La durée de l'opération de cyclisation de l'acide phtalique est le plus souvent comprise entre 1 et 30 minutes.

Les autres caractéristiques de l'opération, telle que par exemple la quantité de terre décolorante super-active, sont celles dictées par le procédé même de cyclisation de l'acide OBB.

La séparation, dans le procédé même de cyclisation de l'acide OBB, de l'acide phtalique sous forme d'anhydride phtalique, et, le cas échéant, de l'anhydride phtalique initialement présent dans l'acide OBB, est économique et d'un intérêt industriel certain. L'anhydride phtalique préexistant et/ou formé à partir d'acide phtalique peut en effet être avantageusement revalorisé par recyclage dans le procédé de synthèse de l'acide OBB, après avoir été récupéré par exemple par condensation selon une technique connue comme la condensation sur une surface dont la température est choisie entre 20° C et 100° C.

Le procédé selon l'invention peut être effectué en discontinu ou en continu.

Un exemple de réalisation continue consiste à remplacer, dans le mode de réalisation continue de la cyclisation de l'acide OBB sur bande transporteuse déjà décrit, l'acide OBB par un acide OBB contenant de l'acide phtalique et, le cas échéant, de l'anhydride phtalique, et à soumettre, sur le parcours de la bande transporteuse, les deux couches superposées de ce produit et de la terre décolorante super-active à un chauffage à température comprise entre 200° C et 250° C de telle façon que l'acide phtalique se cyclise en anhydride phtalique et à ce que l'anhydride phtalique ainsi formé se sublime conjointement à l'anhydride phtalique préexistant le cas échéant dans l'acide OBB pour être recueilli sur la surface d'une bande transporteuse disposée, par rapport à la bande transporteuse principale, comme celle permettant de recueillir l'anthraquinone, et en amont de cette dernière par rapport à l'extrémité de la bande transporteuse principale à laquelle est évacuée la terre décolorante super-active usagée.

Les exemples suivants, donnés à titre non limitatif, illustrent l'invention dans son mode de réalisation en discontinu.

EXEMPLE 1

2,70 g d'acide OBB en poudre sont mélangés grossièrement à froid avec 6,30 9 de "TONSIL" référence BW3. Ce mélange est déposé sous forme d'une couche d'épaisseur pratiquement uniforme de 0,5 cm sur le fond horizontal d'un récipient cylindrique en acier inoxydable de 80 mm de diamètre et de 15 mm de hauteur. Il est recouvert d'une toile fine en acier inoxydable. Un couvercle en acier inoxydable est posé sur le récipient qui est alors porté à 400°C par immersion dans un bain de métal fondu et maintenu à cette température pendant 6 minutes. La température du couvercle est de 100°C environ durant cette opération.

Après enlèvement du récipient du bain de chauffage et refroidissement on recueille sur le couvercle et sur la grille permettant de collecter les cristaux d'anthraquinone pouvant se détacher du couvercle, 2,31 g d'anthraquinone représentant un rendement de cyclisation de l'acide OBB de 93,0%.

EXEMPLE 2

En opérant comme dans l'exemple 1 mais à température de 300°C pendant 1 heure, on recueille 2,26 g d'anthraquinone représentant un rendement de cyclisation de l'acide OBB de 90,8%.

EXEMPLE 3

En procédant comme dans l'exemple 1 mais avec 4,5 g d'acide OBB et 4,5 g de la même terre décolorante super-active on obtient 3,77 g d'anthraquinone représentant un rendement de cyclisation de l'acide OBB de 91,0%.

EXEMPLE 4

Dans le récipient de l'exemple 1 on dépose une couche de 2,70 g d'acide OBB fondu qu'on laisse refroidir et se solidifier à température ambiante avant de déposer sur elle une couche d'épaisseur pratiquement uniforme de 6,5 g de la même terre décolorante que dans l'exemple 1.

En procédant ensuite comme dans l'exemple 1 on recueille 2,35 g d'anthraquinone représentant un rendement de cyclisation de l'acide OBB de 94,7%.

EXEMPLE 5

2,7 g d'un produit contenant 90 % en poids d'acide OBB et 10 % en poids d'acide phtalique sont déposés à l'état fondu sur le fond d'un récipient cylindrique en acier inoxydable de 80 mm de diamètre et de 15 mm de hauteur. Après refroidissement et solidification de la couche ainsi obtenue, on dispose sur elle une couche d'épaisseur pratiquement uniforme de 6,3 g de terre décolorante super-active commercialisée par la Société SÜD-CHEMIE A.G. sous la marque TONSIL, référence BW3.

Le récipient est recouvert d'un couvercle en acier inoxydable et est chauffé à 250°C par immersion dans un bain de métal fondu et est maintenu à cette température durant 6 minutes. La température du couvercle est de 90°C environ durant cette période à la fin de laquelle le couvercle est enlevé et remplacé par un autre qui servira à recueillir l'anthraquinone qui se formera ensuite par cyclisation de l'acide OBB dans les conditions définies dans le brevet principal.

Sur le couvercle enlevé après l'étape à 250°C on recueille 0,18 g d'un sublimat dont l'analyse par chromatographie en couche mince montre qu'il ne contient pratiquement que de l'anhydride phtalique.

EXEMPLE 6 (comparatif)

En procédant comme dans l'exemple 5 mais en l'absence de terre décolorante superactive on ne recueille que 74 % de l'anhydride phtalique récupéré dans l'exemple 5.

EXEMPLE 7

En procédant comme dans l'exemple 5 mais avec un produit renfermant, en poids, 80 % d'acide OBB, 10 % d'acide phtalique et 10 % d'anhydride phtalique, on obtient 0,44 g de sublimat contenant pratiquement que de l'anhydride phtalique.

EXEMPLE 8

En procédant comme dans l'exemple 7 mais en l'absence de terre décolorante super-active, on n'obtient que 61 % de l'anhydride phtalique récupéré dans l'exemple 7.

EXEMPLE 9

En procédant comme dans l'exemple 5 mais avec un produit contenant, en poids, 90 % d'acide OBB, 5 % d'acide phtalique et 5 % d'anhydride phtalique et un chauffage à 250°C d'une durée de 12 minutes, on récupère 0,253 g d'un sublimat qui s'avère encore contenir pratiquement que de l'anhydride phtalique.

**Revendications**

1 - Procédé de cyclisation de l'acide orthobenzoylbenzoïque dans lequel l'anthraquinone formée se sublime, par chauffage de l'acide orthobenzoylbenzoïque en présence d'une terre décolorante super-active constituée d'une argile du type bentonite activée par traitement par un acide minéral, lavée à l'eau de telle façon à ne contenir que 0.1 % au plus d'acide exprimé en acide chlorhydrique et finalement sechée à une température au plus égale à 120°C, à une température comprise entre 300°C et 400°C, caractérisé en ce que l'acide orthobenzoylbenzoïque à l'état solide et la terre décolorante super-active sont mis en présence sous forme d'un mélange de solides disposés en couche d'épaisseur telle que l'anthraquinone formée et sublimée diffuse totalement ou sous forme de deux couches superposées de solides, la couche de terre décolorante super-active recouvrant celle d'acide orthobenzoylbenzoïque de façon à ce que l'anthraquinone formée et sublimée diffuse totalement.

2 - Procédé selon la revendication 1, caractérisé en ce que la terre décolorante super-active renferme, en poids, 70 % à 75 % de SiO₂, 10 % à 20 % de AL₂O₃, 3 % à 5 % de Fe₂O₃, 1 % à 3 % de MgO, 0,5 % à 2 % de CaO, 5 % à 10 % de matières éliminables par calcination, présente une surface spécifique comprise entre 150 m²/g et 200 m²/g et est formée de grains de dimension inférieure à 150 m.

3 - Procédé selon l'une des revendications 1 ou 2, caractérisé en ce que la quantité de terre décolorante super-active est égale 0,5 à 5 parties en poids par partie d'acide orthobenzoylbenzoïque.

4 - Procédé selon l'une quelconque des revendications 1 à 3 caractérisé en ce que la durée de l'opération est comprise entre 5 à 120 minutes.

## Patentansprüche

1. Verfahren zur Cyklisierung von Orthobenzoylbenzoesäure, bei dem das gebildete Antrachinon sublimimiert, durch Erhitzen von Orthobenzoylbenzoesäure bei einer Temperatur zwischen 300 °C und 400 °C in Gegenwart einer superaktiven Bleicherde, aus einem Ton des Bentonittyps, der durch Behandlung mit einer Mineralsäure aktiviert ist, der mit Wasser so weit gewaschen ist, daß er höchstens 0,1 % an Säure, berechnet als Chlorwasserstoffsäure, enthält und der schließlich bei einer Temperatur von höchstens 120 °C getrocknet ist, dadurch gekennzeichnet, daß die feste Orthobenzoylbenzoesäure und die superaktive Bleicherde als Feststoffgemisch in einer Schicht einer derartigen Dicke, daß das gebildete und sublimierte Antrachinon vollständig diffundiert, oder in Form von zwei übereinanderliegenden Feststoffschichten eingesetzt werden, wobei die Schicht der superaktiven Bleicherde die der Orthobenzoylbenzoesäure derart bedeckt, daß das gebildete und sublimierte Antrachinon vollständig diffundiert.

2. Verfahren nach Anspruch 1, dadurch gekennzeichnet, daß die superaktive Bleicherde in Gewichtsprozent 70 % bis 75 % SiO₂, 10 % bis 20 % Al₂O₃, 3 % bis 5 % Fe₂O₃, 1 % bis 3 % MgO, 0,5 % bis 2 % CaO und 5 % bis 10 % an Bestandteilen enthält, die beim Kalzinieren entfernt werden können, eine spezifische Oberfläche zwischen 150 m²/g und 200 m²/g besitzt und in einer Korngröße unter 150 µm vorliegt.

3. Verfahren nach einem der Ansprüche 1 oder 2, dadurch gekennzeichnet, daß die Menge der superaktiven Bleicherde 0,5 bis 5 Gewichtsteile je Gewichtsteil Orthobenzoylbenzoesäure beträgt.

4. Verfahren nach einem der Ansprüche 1 bis 3, dadurch gekennzeichnet, daß die Dauer der Behandlung zwischen 5 und 120 Minuten beträgt.

## Claims

1. Process for cyclization of ortho-benzoylbenzoic acid in which the anthraquinone formed sublimes, by heating ortho-benzoylbenzoic acid in the presence of a super-active bleaching clay consisting of a clay of the bentonite type activated by treatment with an inorganic acid, washed with water so that it only contains 0.1% at most of acid, expressed as hydrochloric acid, and finally dried at a temperature equal at most to 120°C, at a temperature of between 300°C and 400°C, characterized in that orthobenzoylbenzoic acid in the solid state and the super-active bleaching clay are brought into contact in the form of a mixture of solids arranged in a layer of thickness such that the anthraquinone formed and sublimed diffuses completely, or in the form of two superposed layers of solids, the super-active bleaching clay layer covering that of ortho-benzoylbenzoic acid so that the anthraquinone formed and sublimed diffuses completely.

2. Process according to Claim 1, characterized in that the super-active bleaching clay contains, by weight, 70% to 75% of SiO₂, 10% to 20% of Al₂O₃, 3% to 5% of Fe₂O₃, 1% to 3% of MgO, 0.5% to 2% of CaO and 5% to 10% of matter removable by calcination, has a specific surface area of between 150 m²/g and 200m²/g and is composed of particles of size less than 150 µm.

3. Process according to one of Claims 1 or 2, characterized in that the amount of super-active bleaching clay is equal to 0.5 to 5 parts by weight per part of ortho-benzoylbenzoic acid.

4. Process according to any one of Claims 1 to 3, characterized in that the duration of the operation is between 5 and 120 minutes.